(19)

Europäisches Patentamt
European Patent Office
Office européen des brevets

(11) **EP 1 471 939 B1**

(12) **EUROPEAN PATENT SPECIFICATION**

(45) Date of publication and mention of the grant of the patent:
**18.08.2010 Bulletin 2010/33**

(21) Application number: **02805842.8**

(22) Date of filing: **24.12.2002**

(51) Int Cl.:
**A61K 41/00** (2006.01)

(86) International application number:
**PCT/GB2002/005927**

(87) International publication number:
**WO 2003/055520 (10.07.2003 Gazette 2003/28)**

(54) **DELIVERY OF NEUTRON CAPTURE ELEMENTS FOR NEUTRON CAPTURE THERAPY**

ZUFÜHRUNG VON NEUTRONENEINFANGELEMENTEN ZUR NEUTRONENEINFANGTHERAPIE

ADMINISTRATION D'ELEMENTS DE CAPTURE DE NEUTRONS DESTINES A UNE THERAPIE PAR CAPTURE DE NEUTRONS

(84) Designated Contracting States:
**AT BE BG CH CY CZ DE DK EE ES FI FR GB GR IE IT LI LU MC NL PT SE SI SK TR**

(30) Priority: **28.12.2001 GB 0131058**

(43) Date of publication of application:
**03.11.2004 Bulletin 2004/45**

(73) Proprietor: **Psimei Pharmaceuticals Plc Guildford, Surrey GU1 3DG (GB)**

(72) Inventor: **PATEL, Bipin, C. M., Psimei Pharmaceuticals plc Guildford, Surrey GU1 3DG (GB)**

(74) Representative: **Hart, Deborah Mary et al Kilburn & Strode LLP 20 Red Lion Street London WC1R 4PJ (GB)**

(56) References cited:
WO-A-00/44682    WO-A-00/45826
WO-A-01/34196    US-A- 5 443 813

- TOKUMITSU H ET AL: "CHITOSAN-GADOPENTETIC ACID COMPLEX NANOPARTICLES FOR GADOLINIUM NEUTRON-CAPTURE THERAPY OF CANCER: PREPARATION BY NOVEL EMULSION-DROPLET COALESCENCE TECHNIQUE AND CHARACTERIZATION" PHARMACEUTICAL RESEARCH, NEW YORK, NY, US, vol. 16, no. 12, 1999, pages 1830-1835, XP000951445 ISSN: 0724-8741
- LAWACZECK R ET AL: "GADOLINIUM NEUTRON CAPTURE THERAPY (GDNCT) WITH MRI CONTRAST MEDIA IN VITRO STUDIES" CANCER NEUTRON CAPTURE THERAPY, XX, XX, 1996, pages 859-864, XP000951499
- DATABASE CA [Online] CHEMICAL ABSTRACTS SERVICE, COLUMBUS, OHIO, US; FUKUMORI, YOSHINOBU ET AL: "Nano-particulate design and preparation for targeting and controled release of drugs" retrieved from STN Database accession no. 135:127058 XP002224876 & INTERNATIONAL CONFERENCE ON PROCESSING MATERIALS FOR PROPERTIES, PROCEEDINGS, 2ND, SAN FRANCISCO, CA, UNITED STATES, NOV. 5-8, 2000 (2000) 453-458. EDITOR(S): MISHRA, BRAJENDRA;YAMAUCHI, CHIKABUMI. PUBLISHER: MINERALS, METALS & MATERIALS SOCIETY, WAR,
- THOMAS J ET AL: "Dodeca (carboranyl)-substituted closomers: toward unimolecular nanoparticles as delivery vehicles for BNCT." CHEM COMMUN (CAMB), (2001 SEP 21) (18) 1884-5., XP002224874

EP 1 471 939 B1

**(Cont. next page)**

- **ALI O. SEZER ET AL.: "Chemical vapor deposition of boron carbide." MATERIALS SCIENCE AND ENGINEERING, vol. B79, 2001, pages 191-202, XP002224875**
- **MACPHERSON M S ET AL: "DOSE DISTRIBUTIONS AND DOSE RATE CONSTANTS FOR NEW YTTERBIUM-169 BRACHYTHERAPY SEEDS" MEDICAL PHYSICS, AMERICAN INSTITUTE OF PHYSICS. NEW YORK, US, vol. 22, no. 1, 1995, pages 89-96, XP000505147 ISSN: 0094-2405**
- **CHENG ET AL: "Neutron-activatable glass seeds for brachytherapy" JOURNAL OF NUCLEAR MEDICINE, SOCIETY OF NUCLEAR MEDICINE. NEW YORK, US, vol. 35, no. 5, 5 June 1994 (1994-06-05), page 242P XP002111090 ISSN: 0161-5505**

**Description**

The invention relates to neutron capture therapy and, in particular, to new ways of delivering neutron capture elements to a desired target site.

The theoretical principle of neutron capture therapy (NCT) for the treatment for cancer was first described in 1936 by the American scientist Locker. In essence, when a stable isotope such as Boron-10 is irradiated by non-ionizing slow neutrons, a fission reaction results that leads to the emission of highly ionizing radiation with a range of between $7\mu m$ and $9\mu m$. Thus, when $^{10}B$ is irradiated in this way inside e.g. a tumour cell, the range of the resulting ionising radiation is sufficient to destroy that cell but not surrounding tissue. In 1951 the first patient was treated. Since then the principle has been successfully demonstrated in the clinic.

The level of success has varied and has been dependent on a combination of the quality of neutrons and the pharmaceutical properties of the neutron capture element carrier.

NCT is a bimodal therapy requiring spatial and temporal overlap of neutrons and the drug. To achieve a biological effect, an interaction between slow neutrons and e.g. a boron carrying agent are necessary. The NCT reaction of $^{10}B$ with neutrons (BNCT) may be summarised by the following equation:

$$^{10}B + {}^{1}n_{th} \rightarrow {}^{7}Li + \alpha \text{ particle } ({}^{4}He) + 2.4 \text{ MeV}$$

The 2.4 MeV energy is taken up as kinetic energy by the $Li^{+}$ and $He^{2+}$ ions. The two particles are sufficiently energetic to generate intense ionization tracks with a maximum range of 9 $\mu$m. In this way, the damage is confined to the diameter of a tumour cell i.e. about $10\mu m$. Thus, only the cells that contain boron are damaged, while non-boron containing healthy cells are left intact. The isotope Boron-10 is stable and non-radioactive and its nucleus has a very large neutron absorption cross section for slow neutrons, 2,700 times greater than that for hydrogen. This translates into the ability to absorb neutrons of comparatively several thousand times better than that of the elements constituting living tissues (such as hydrogen, oxygen, carbon).

NCT can be used to treat cancers which are normally treated with radiotherapy such as lymphomas and skin cancers, as well as cancers of the brain, breast, lung, head and neck, bone, prostate, pancreas and cervix. In addition when surgical removal of a tumour is planned, NCT may also be used to help shrink the size of the tumour and to reduce the associated normal tissue loss.

NCT has been driven by the potential benefits of selective "in situ" radiotherapy and as a potential substitute to conventional x-ray radiotherapy. Over 500 patients have received experimental BNCT treatment for brain and skin tumours World-wide to date.

NCT is advantageous over conventional radiotherapy in a number of respects. Thus, in conventional radiotherapy the biological effect is spread over the entire irradiated area whilst with NCT it is specific to those cells containing neutron capture element carrier molecules. A relatively high radiation dose is required with radiotherapy to generate the destructive ionisation tracks for the biological effect. This is a limiting factor in the effectiveness of the treatment. Radiotherapy is further limited by the inherent nature of the low LET (linear energy transfer) radiation beam which comprises electrons (β-particles) or photons (X-rays and γ-rays). With BNCT short range high energy α alpha and $^{7}Li$ particles are generated. These particles are high LET particles that are intensely ionizing and exhibit a greater, more potent, destructive propensity. Hence, the BNCT dose required to generate an equivalent radiotherapy biological effect is very much smaller than the relative radiotherapy dose.

Radiotherapy is widely used in the management and treatment of cancers where it exhibits a 30% cure rate expected to increase to 33% over 10 years, with 70% palliation. However, radiotherapy results in radiation damage to normal tissue and typically requires a 6 week treatment schedule.

In contrast to radiotherapy NCT offers target tissue selectivity via the specificity of the drug. Lethal radiation is only generated where the neutron capture element is localised i.e. within the tumour tissue. Thus, for example, in BNCT the radiation is generated in boron containing tumour cells, and damage is localised to those cells. This is a key advantage of NCT over radiotherapy to potential destroy tumours that are normally not clearly demarcated, but are diffuse.

NCT is not dependent up on oxygen levels in the tumour, and this is an advantage as many tumours are hypoxic. Moreover, NCT can also be used where the cancer treatment is anatomically compromised. Deep seated tumours can be treated, for example, from 4cm-8cm depth and beyond. Additionally, NCT is less demanding for the patient than conventional radiotherapy as it can be given several times over a period of 2-4 days. By contrast, conventional radiotherapy needs to be given up to 30 times over a period of six weeks.

A further advantage of NCT is that in such a binary system each component can be manipulated independently of the other. The interval between administration of the, e.g. $^{10}B$ agent and neutron irradiation can be adjusted to an optimum time when there is the highest differential $^{10}B$ concentrations between normal tissues and the tumour tissue, Similarly, the neutron beam can be collimated so that the field of irradiation is limited to the tumour site, and normal tissues with a residual $^{10}$B concentration can be excluded from the treatment volumes.

In summary the benefits of NCT are as follows:

- NCT is a biomodal therapy -time interval between administration of drug and the time of "drug-activation" can be optimised to attain maximum tumour kill
- Selective targeting of tumour cells and sparing of normal tissues
- Radiation generated and localised only in those cells with sufficient quantity of the neutron capture element carrying agent
- Effective for diffuse spread and potential to target distant single tumour cells or metastases
- NCT is applicable to radioresistant tumours and anatomically compromised sites
- NCT is not dependent on oxygen content in tumours
- NCT can access over 4cm- 8cm of tumour depth, there is effectively no limitations to the depth of a tumour
- Treatment time of 2-4 days compared to typical 6 weeks with radiotherapy

To reap the potential benefits of NCT as an "in situ" cellular radiotherapy it is a preferable that:

(i) The neutrons are of an energy range where only the neutron capture element atoms are able to undergo a fission reaction;

(ii) The, e.g. boron atoms or the NCT agent is selectively localised in the target tissue;

(iii) For BNCT, there is optimal concentration of Boron drug in the target tissue. For example $15\mu g$-$35\mu g$ / g of tumour tissue (equating to $10^9$ boron atoms) is widely quoted to be the amount of $^{10}$B necessary for BNCT using a neutron beam from a nuclear reactor of fluence $10^9$ neutrons per second per $cm^2$;

(iv) The neutron capture element drug should have an unusually low toxicity compared to conventional pharmaceutical agents since large concentration of the compound need to be administered; and

(v) Sufficiently rapid clearance from the blood so that there is a very high ratio of tumour to blood concentration, and a very high tumour to normal tissue concentration.

To date, predominantly two experimental compounds, 4-dihydroxyborylphenylalanine (BPA) and sodium mercaptoundecahydrododecaborate (BSH) have been used to deliver Boron as the neutron capture element in clinical trials.

The following table (Table 1) shows some characteristics of Boron neutron capture therapy agents that are currently being evaluated.

Table 1

| Compound | Absolute tumour concentration (ug/g) | Tumour:blood ratio | Tumour:CNS Ratio |
| --- | --- | --- | --- |
| BPA (in clinical trials | 16.5 | 2.2: 1.0 | 1.7 :1.0 |
| BSH (in clinical trials) | 6.9 | 0.7 : 1.0 | 3.0: 1.0 |
| CuTCPH (preclinical development) | 114.0 | 570:1.0 | 57:1.0 - |

As noted above, a requirement of NCT is that the neutron capture element is targeted specifically or predominantly to the tissue site of interest. This mission critical criterion has yet to be fully satisfied by compounds in clinical trials to date, due to the lack of specificity and low target tissue to normal tissue distribution ratios observed. As a result the clinical development and wide spread use of BNCT has been severely hindered. Thus far, only the CuTCPH compound developed by Michiko Mura appears to provide adequate boron localisation.

To date neutron capture agents have been targeted to tumors by various drugs. Boron has been incorporated into a huge range of drug molecules including protein targeting systems whose toxicity, pharmacokinetics, biodistribution and radiobiology have been studied, and in some promising instances attempts have been made to optimise these characteristics via formulation in co-solvents and drug delivery systems. However, from over 50 years of research and development effort only two sub-optimal compounds (BOA and BSH) have entered clinical trials. These compounds have low toxicity but suffer from poor selectivity for tumors and inadequate pharmacokinetics suitable for routine BNCT therapy. A more detailed review of Boron drug candidates studied in the prior art is set out in Table 2.

Table 2

| | Boron drug candidate | Issue | Reference |
|---|---|---|---|
| 1. | Boronated amino acids Eg. BPA Boronophenylalanine Boronated amino acids and peptides | Boronated phenylalanine is in clinical trials. It is not selective and the kinetics are such that it need to be given several times over a periods of day. A major problem is that it needs to be given in large volumes such as 1,000ml by intravenous injection. This causes haemodynamic disturbance which poorly tolerated by patients whom are already seriously ill. | Snyder H R et al (1958)J Am Chem Soc 80, 835. See pages 1531-1534 Soloway (1998) Chem Rev. |
| 2. | Modified carborane cage Eg. $[B_{10}H_{10}]^{2-}$ decahydrodecaborate Eg. $[B_{12}H_{12}]^{2-}$ Dodecahydrodecaborate Eg. $C_2B_{12}H_{12}$ | Not very effective as standalone but are used as boron carries for attachment to other molecules. | Hawthorn, M F et al (1959) J Am Chem Soc 81, 5519. Grimes R, N. Carboranes. Academic press. NY 1970. |
| 3. | $B_{12}H_{12}$ SH$^{2-}$ Mercaptoundecahydrododecaborate (BSH) May also be in the form or a dimer "BSSB" Is a second generation BNCT compound | In clinical trials, not very selective, inappropriate kinetics, several doses need to be given. | Soloway A H et al J Med Chem 1967, 10. 714. |
| 5 | Porphyrins and Phthalocyanines Eg. BOPP, Eg. CuTCPH | Porphyrins carry 4 carborane cages. BOPP as been shown to have phototoxicity and poor selectivity for tumours. CuTCPH appears toto show favorable tumourstumour :blood: normal tissue distribution. | Ozawa T., Pro Am Ass for Cancer March 1998, 39, p586 Miura M.2001, Radiation research 155, 603-610. |
| 6. | Boron containing Nucleic acid precursors Boronated pyrimidines and purines 5-(dihydroxyboryl) uracil 5-carboranyluracil carborane containing pyrimidines (these are considered as third generation compounds) | Targeted as precursors for DNA and RNA synthesis, failed to show any promise. | Liao. T.K, J Am Chem Soc 1964, 86. 1869. Schinazi R, F J Org. Chem. Soc 1985, 50, 841. Nemoto. H. J Chem Soc. Chem Commun. 1994, 577. |

(continued)

| | Boron drug candidate | Issue | Reference |
|---|---|---|---|
| 7. | Proteins<br><br>Antibody -boron conjugates | A two step bispecific antibody system is described where the boron is attached to the antibody via boronated dextran. | Griffiths G L et al. US patent 5,846,741 (1998) |
| 8. | DNA binders<br>Alkylating agents, interchalators<br>-acridines and tetracycline's,<br>phenanthridine Groove binders<br>-carborane derivatives | Have been synthesised but none has yet shown any promise. | Soloway 1998 Chemical reviews Vol 98 No.4 p1538-1541. |
| 9. | Gd-157 agent a combined BNCT and GdNCT compound | The Gd and a carborane cage are on the same molecule. | Soloway 1998 Chemical reviews Vol 98 No.4 p1519. |
| 9. | Lipids and Liposomes<br><br><br><br>B-lipiodol | Used for encapsulating lipid soluble and water soluble compounds, BNCT compound delivery systems. | Soloway 1998 Chemical reviews Vol 98 No.4 p1533-1534.<br>Shelly K Proc Natl Acad Sci USA 1992,89.9039.<br>Chou FI, Anticancer Res 1999 May-Jun;19(3A):1759-64 |
| 10 | Others including, foliates growth factors, hormones, radiation sensitisers, phosphates, phosphonates and phosphoramidates, cyclic thiourea derivatives, amines, promazines, hyantoins, barbiturates | Many classes of these compounds have been synthesised and none have so far made it to the clinic. | Soloway 1998 Chemical reviews Vol 98 No.4 p1545-1550. |

All of the above are conventional drug candidates or biopharmaceuticals that are dissolved in either an aqueous solution and/or in an organic co-solvent system that uses standard pharmaceutical excipients such as oils, alcohol's, ethers or lipids. These formulations are then administered via routes such as oral, intravenous, intraperitonial, intramuscular, dermal and colonic. Almost always these dissolved compounds and on occasion co-solvents have shown adverse systemic toxicity and pharmacological/ metabolic breakdown activity profile (anaphylaxis, pain at the site of injection, emboli formation, and precipitation). The drug candidate molecules have one or more $^{10}$B atoms incorporated into their structure via covalent bonding. Normally, carborane cages are used to add as many boron's as is possible to the central "carrier" molecule.

There is a need for improved neutron capture element carriers capable of selectively delivering the neutron capture element (for example, boron) to a target tissue. Such carriers should exhibit low toxicity and appropriate clearance kinetics, and ideally should be capable of the delivery of large doses of neutron capture elements to tumour tissue. The current invention sets out to provide such a carrier.

Thus, in a first aspect, the invention provides water insoluble nanoparticles comprising at least one neutron capture element in an inorganic form for use in therapy, surgery or diagnosis wherein said at least one neutron capture element is selected from the group consisting of boron, preferably $^{10}$B, said nanoparticle comprising a biocompatible outer layer selected from polyvinylpyrrolidine (PVP). In a second aspect, the invention provides a pharmaceutical composition comprising a water insoluble nanoparticles comprising at least one neutron capture element in an inorganic form as defined in claim 1.

Any form of nanoparticle may be used in the invention, as long as it is water insoluble. Thus, although the term nanoparticle is sometimes used to describe colloidal particles consisting of macromolecular substances (whether artificial or natural), and often includes lyposomes, proteins, peptides, strands of DNA and RNA, and polymers, only those nanoparticles that are water insoluble are intended to be covered. Thus, the present invention utilises materials that are insoluble in aqueous media and, in general, in all conventional pharmaceutical solvents.

The at least one neutron capture element is present in the water insoluble nanoparticle in an inorganic form. The neutron capture element is boron and, in particular, $^{10}$B.

The neutron capture element may be present in a natural crystalline form, or in a particulate form. Alternatively, the neutron capture element may be in the form of a glass or glass ceramic, in the form of a polymerised inorganic matrix or in the form of a sol-gel derived xerogel. In some embodiments, the neutron capture element may also be in the form of an organically modified ceramic, for example, wherein the element comprises at least one bond to a hydrocarbon chain. Such organically modified ceramics behave as inorganic materials because the metal-oxygen-metal system dominates over the few metal-carbon bonds. Such organically modified ceramics maintain the neutron capture element in a system that is inert and insoluble in water.

Nanoparticles of the embodiments of the invention further comprise a biocompatible outer layer which is selected from the group consisting of polyvinylpyrrolidine (PVP). Such hydrophilic biocompatible outer layers impart the function of stealth to the nanoparticles, allowing them to escape detection by the reticuloendothelial system of the liver, spleen and pancreas. The outer covering also acts as a surfactant and functions to prevent aggregation of the particles.

These biocompatible layers are adsorbed onto the core structure and do not necessarily chemically react to the surface. Preferably, the outer layer does not include intramolecular cross-linkages.

In an alternative embodiment, the neutron capture element can be present as a layer or film around an inorganic nanoparticle core such as mica, xeolites, $TiO_2$ spheres, $ZrO_2$ spheres or particles, or organic polymer particles or spheres.

In a further embodiment, nanoparticles of the invention may further comprise a pharmacologically active substance which may, or may not be loaded into the nanoparticles by absorption, adsorption or incorporation. In preferred embodiments, the pharmacologically active substance is a chemotherapeutic agent.

In some embodiments, nanoparticles of the invention further comprise a metal selected from the group consisting of vanadium (V), manganese (Mn), iron (Fe), ruthenium (Ru), technetium (Tc), chromium (Cr), platinum (Pt), cobalt, (Co), nickel (Ni), copper (Cu), zinc (Zn), germanium, (Ge), indium (In), tin (Sn), yttrium (Y), gold (Au), barium (Ba), tungsten (W), and gadolinium (Gd). In some embodiments, the further metal is present at a concentration of about 0.0001 % wt/wt to about 0.1% wt/wt.

In a third aspect, the invention provides the use of water insoluble nanoparticles comprising at least one neutron capture element in an inorganic form as defined in claim 1 in the manufacture of a medicament for use in neutron capture therapy.

In some embodiments of any of the above uses, the neutron capture therapy is for the treatment of ablation of cancer or other diseased tissues, for example, lymphomas, skin cancer, breast cancer, lung cancer, head and neck cancer, bone cancer, prostate cancer, cancer of the pancreas, cervical cancer, brain cancers e.g. glioblastomas, primary and secondary metastases and benign and metastatic prostate cancers, e.g. benign prostate hyperplasia. In some embodiments, the tumours to be treated are solid and discrete. In treatment methods of the invention, neutron capture therapy can be administered over a period of e.g. 1 to 14 days. The therapeutic methods of the invention may further comprise the step of removing a tumour by surgery. In a further embodiment, the method of the invention comprises analysing the concentration of the neutron capture element in the target tissue, for example, using MRI, PET or SPECT imaging.

For dosemetry it is necessary to calculate the effective therapeutic BNCT radiation dose so as to provide the optimal treatment protocol. For this, knowledge of the NCT compound concentration in the target tissue is required. Conventionally, blood concentration or ideally the target tissue concentrations are derived using invasive techniques. The invention allows the incorporation of Magnetic Resonance Imaging MRI contrast agents such as Gadolinium or radionucelotides such as $^{67}$Cu and Tc-99m-HMPAO for Positron Emission Tomography (PET) and Single photon emission computed tomography (SPECT) respectively. MRI provides vital anatomical diagnostic information in addition to the NCT compound concentration. PET, allows the examination of the heart, brain, and other organs and images show the chemical functioning of an organ or tissue and is particularly useful for the detection of cancer, coronary artery disease and brain disease, unlike X-ray, CT, or MRI which show anatomical structure. Single-photon emission computed tomography (SPECT), like PET acquires information on the concentration of radionucleotides.

In a fourth aspect, the invention provides a process for the preparation of water insoluble nanoparticles comprising at least one neutron capture element in an inorganic form as defined in claim 1, said process comprising (i) providing at least a first mass of said neutron capture element in an inorganic form; (ii) providing at least a second mass of the same type of material; (iii) mixing said first and second masses in the absence of other abrasive material; (iv) causing frictional abrasion between said first and second masses; and (v) collecting said nanoparticles.

In some embodiments of this aspect of the invention, at least one further, non-abrasive material is included in the mixing step, and the non-abrasive material may be selected from the group consisting of vanadium (V), manganese (Mn), iron (Fe), ruthenium (Ru), technetium (Tc), chromium (Cr), platinum (Pt), cobalt, (Co), nickel (Ni), copper (Cu), zinc (Zn), germanium, (Ge), indium (In), tin (Sn), yttrium (Y), gold (Au), barium (Ba), tungsten (W), and gadolinium (Gd).

The invention is now described in more detail with reference to the following drawings in which:-

Figure 1 shows a first and second embodiment of an insoluble inorganic nanoparticle.
Figure 2 shows a third embodiment of an insoluble inorganic nanoparticle.
Figure 3 shows a fourth embodiment of an insoluble inorganic nanoparticle.
Figure 4 shows various embodiments of insoluble inorganic nanoparticles having biocompatible outer stealth layers.

In one embodiment the invention utilizes angstrom (Å) sized particles that are totally insoluble solid inorganic materials that may have their surface properties modified for optimal pharmacokinetics and minimal toxicity for neutron capture therapy in the treatment of, for example, various forms of cancer.

The current invention overcomes the problems of cytotoxicity and the deposition of a high amount of neutron capture element at the tumour site and minimises the amount of neutron capture element in normal tissues. The invention provides for rapid clearance from the blood and further provides a diagnostic option to image tumour location and size and to pick up distant metastases.

In a preferred embodiment, the invention involves the direct use of $^{10}$B as a metal, and inorganic materials such as Boron-transition metal complexes, boron oxides, boron nitrides, boron carbides and large carbonate cages. The physical form of these materials may be angstrom sized particles or engineered angstrom sized particles which may be coated with a biocompatible surface modifier e.g. a surfactant. Alternative engineered particles comprise an organic polymer or an inorganic non-NCT element containing core covered with a $^{10}$B coating and optionally covered with a biocompatible surfactant. Further alternative engineered particles comprise a $^{10}$B inorganic or mostly inorganic core or a Boron containing polymer core that is covered by a chemically and mechanically protective coating, for example a metal oxide coating, that is optionally further covered with a biocompatible surfactant

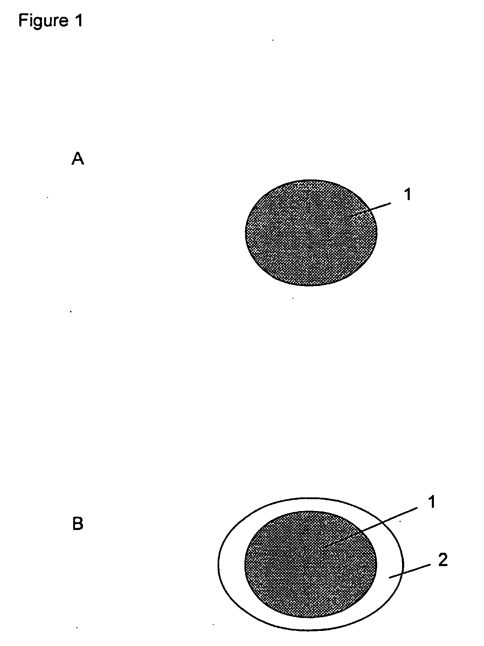

Figure 1 shows the basic form of the inorganic, insoluble nanoparticles of some embodiments of the invention. The nanoparticle 1 consists of pure NCT metal, such as $^{10}$B glass or ceramic and has a size of between about 1 and 10,000 Å ($10^{-10}$ to $10^{-6}$ m). Preferably, the nanoparticles are between about 1 and 1,000 Å ($10^{-10}$ to $10^{-7}$ m), more preferably between about 1 and 100 Å ($10^{-10}$ to $10^{-8}$ m)and ideally between about 10 and 100 Å ($10^{-9}$ to $10^{-8}$ m).

Figure 1b shows an alternative embodiment, in which the pure NCT metal particle 1 is surrounded by a chemically and/or mechanically protective coating, 2, for example, $TiO_2$. In each case, the neutron capture element of nanoparticle 1 can be in the form of, for example, a metal compound, a glass, ceramic, crystal, ormacil or xerogel. Preferably, the neutron capture element is Boron, although other elements are useful for neutron capture element as discussed above.

The physical form of the inorganic NCT agent may be its natural crystalline form or in the form of particles, or spheres or polymeric structures. This will depend up on the manufacturing route. Physical properties of the angstrom sized nanoparticles particles can also be those typically characterised by a Glass, (a supper cooled liquid, that is an amorphous randomly organised molecular structure which does not produce an x-ray diffraction pattern), a ceramic -crystalline phase or glass-ceramic (a structure that exhibits characteristics of a glass that has a crystalline phase). Alternatively, the nanoparticles may be sol-gel derived materials (polymerised inorganic matrix ~ metal-oxygen-metal), sol-gel derived xerogels (partially or fully condensed material), or an organically modified ceramic where the metal has one or more M-R bonds where R is a hydrocarbon chain. These materials are chemically stable and totally insoluble in aqueous media although they may react with water producing oxides but do not dissolve. The NCT materials do not dissolved in water even at elevated temperatures. Silica glasses.are amongst the most chemically inert of commercial materials. They react with almost no liquids or gases at low temperatures (~300C) and at higher temperatures they only react with gaseous HF. In some embodiments, nanoparticles of the invention cannot be detected after about 2 to about 12 hours in the blood plasma.

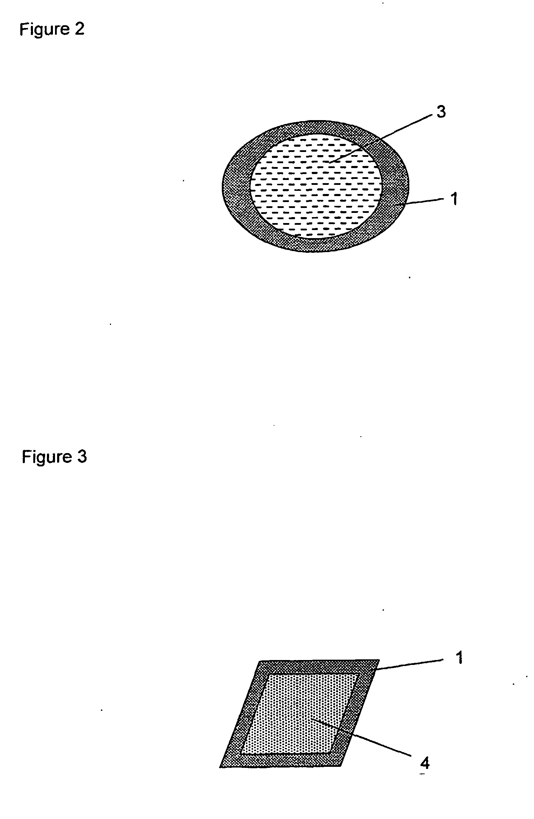

Figure 2 shows an alternative embodiment of the invention, in which the neutron capture element, for example, Boron, is present as a thin film surrounding an inorganic or organic polymer core, and Figure 3 shows a further alternative embodiment in which the neutron capture element 1 is present as a thin film surrounding an inorganic or organic crystalline core which may or may not contain the neutron capture therapy element. Neutron capture element layer 1 has a thickness of anything from a few atoms to tens of atoms, and the core can be, for example, mica, zeolites, $TiO_2$ spheres, $ZrO_2$ spheres or particles or organic polymer particles or spheres.

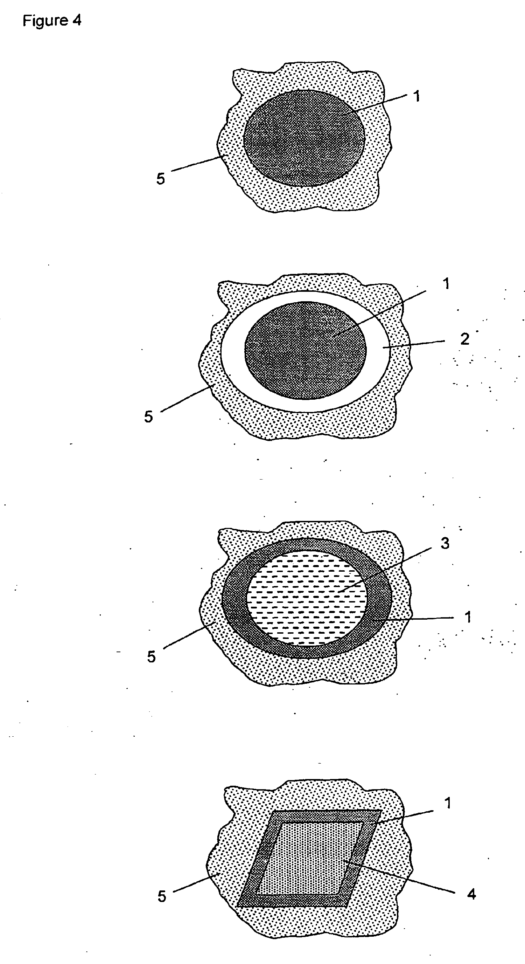

Figure 4 shows the various embodiments of Figures 1 to 3 modified by the addition of an outer biocompatible stealth layer 5. The biocompatible stealth layers are adsorbed onto the core structure. They do not necessarily chemically react to the surface and preferably do not have any intermolecular cross-linkage. The layers are selected from a group consisting of polyvinypyrrolidone(PVP).

In some embodiments, particles of the invention may be loading with a pharmacologically active substance by absorption, adsorption, or incorporation to impart a duel therapeutic effect of chemotherapy and NCT. The raw particles may be coated with a surfactant as noted above directly in the presence or in the absence of a loaded pharmaceutical agent. Chemotherapeutic agents my be loaded to deliver a double blow to a cancer cell in the form of an instantaneous combination therapy. Suitable chemotherapeutic agents include: melphalan, cyclophospamide, doxorubicin carmusline, methotrexate, 5-fluorouracil, cytrabine, mercaptopurine, anthracyclines, daunorubicin, epirubicin, vinca alkaloids, mitomycin C, vinblastin, vincristine, dactinomycin, taxol, Lasparaginase; G-CSF, cicplatin, carboplatin.

Other drugs and their prospective prodrugs which may be incorporated into nanoparticles of some embodiments include any one of the following:

1.    5-azurudub-I-yl)-4-dihydroxylamino-2-nitrobenzamide

(continued)

2. Gancyclovie triphosphate
3. Phenylenediamine mustard
4. Benzoic acidic mustards
5. Adenine arabinonucleoside triphophate (araATP)
6. Phenylenediamine mustard
7. Hydrogen peroxide
8. Superoxide, hydrogen peroxide
9. Methotrexate
10. Phenylenediamine mustards
11. Cyanide
12. Etoposide
13. Topoisomerage inhibitors such as camptothecin and topotecan
14. Mitomycin alcohol
15. Palytoxion
16. Melphalan
17. Taxanes, such as taxol and taxotere
18. 5-(aziridin-1-yl)-4-hydroxylamino-2-nitrobenzamide
19. Actinomycin D, Mitomycin C

The insoluble nanoparticles of the invention can be formulated into pharmaceutical compositions for the treatment, by neutron capture therapy of a number of tumours and other tissue disorders. Treatment involves the administration of an effective dose to a patient, followed by irradiation with neutrons.

The determination of an effective dose is well within the capability of those skilled in the art. For any NCT compound, the therapeutically effective dose can be estimated initially either in cell culture assays or in an appropriate animal model. The animal model is also used to achieve a desirable concentration range and route of administration. Such information can then be used to determine useful doses and routes for administration in humans.

A therapeutically effective dose refers to that amount of active agent which ameliorates the symptoms or condition. Therapeutic efficacy and toxicity of such compounds can be determined by standard pharmaceutical procedures in cell cultures or experimental animals (e.g., $ED_{50}$, the dose therapeutically effective in 50% of the population; and $LD_{50}$, the dose lethal to 50% of the population). The dose ratio between therapeutic and toxic effects is the therapeutic index, and it can be expressed as the ratio, $LD_{50}/ED_{50}$. Pharmaceutical compositions which exhibit large therapeutic indices are preferred. The data obtained from cell culture assays and animal studies is used in formulating a range of dosage for human use. The dosage of such compounds lies preferably within a range of circulating concentrations that include the $ED_{50}$ with little or no toxicity. The dosage varies within this range depending upon the dosage form employed, sensitivity of the patient, and the route of administration.

The exact dosage may be chosen by the individual physician in view of the patient to be treated. Dosage and administration can be adjusted to provide sufficient levels of the active moiety or to maintain the desired effect. Additional factors which may be taken into account include the severity of the disease state (e.g. tumour size and location); age, weight and gender of the patient; diet; time and frequency of administration; drug combination(s); reaction sensitivities; and tolerance/response to therapy.

Administration of pharmaceutical compositions of the invention may be accomplished orally or parenterally. Methods of parenteral delivery include topical, intra-arterial, intramuscular, subcutaneous, intramedullary, intrathecal, intraventricular, intravenous, intraperitoneal, or intranasal administration. In addition to the neutron capture element nanoparticles and optional active ingredients, these pharmaceutical compositions can contain suitable pharmaceutically acceptable carriers comprising excipients and other compounds that facilitate processing of the active compounds into preparations which can be used pharmaceutically. Further details on techniques for formulation and administration can be found in the latest edition of Remington's Pharmaceutical Sciences (Maack Publishing Co, Easton PA).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, etc, suitable for ingestion by the patient.

Pharmaceutical preparations for oral use can be obtained through combination of active compounds with solid excipient, optionally grinding a resulting mixture, and processing the mixture of granules, after adding suitable additional compounds, if desired, to obtain tablets or dragee cores. Suitable excipients are carbohydrate or protein fillers include, but are not limited to sugars, including lactose, sucrose, mannitol, or sorbitol; starch from corn, wheat, rice, potato, or other plants; cellulose such as methyl cellulose, hydroxypropylmethyl-cellulose, or sodium carboxymethylcellulose; and gums including arabic and tragacanth; as well as proteins such as gelatin and collagen. If desired, disintegrating or solubilizing agents may be added, such as the cross-linked polyvinyl pyrrolidone, agar, alginic acid, or a salt thereof, such as sodium alginate.

Dragee cores can be provided with suitable coatings such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterise the quantity of active compound (i.e. dosage).

Pharmaceutical preparations which can be used orally include push-fit capsules made of gelatin, as well as soft, sealed capsules made of gelatin and a coating such as glycerol or sorbitol. Push-fit capsules can contain active ingredients mixed with a filler or binders such as lactose or.starches, lubricants such as talc or magnesium stearate, and, optionally, stabilizers. In soft capsules, the active compounds can be dissolved or suspended in suitable liquids, such as fatty oils, liquid paraffin, or liquid polyethylene glycol with or without stabilizers.

Pharmaceutical formulations for parenteral administration include aqueous suspensions of NCT nanoparticles of the invention. For injection, the pharmaceutical compositions of the invention may be formulated in aqueous solutions, preferably in physiologically compatible buffers such as Hanks's solution, Ringer's solution, or physiologically buffered saline. Aqueous injection suspensions can contain substances which increase the viscosity of the suspension, such as sodium carboxymethyl cellulose, sorbitol, or dextran. Additionally, suspensions of the nanoparticles can be prepared as appropriate oily injection suspensions. Suitable lipophilic vehicles include fatty oils such as sesame oil, or synthetic fatty acid esters, such as ethyl oleate or triglycerides, or liposomes.

For topical or nasal administration, penetrants appropriate to the particular barrier to be permeated are used in the formulation. Such penetrants are generally known in the art.

The pharmaceutical compositions of the present invention can be manufactured in substantial accordance with standard manufacturing procedures known in the art (e.g. by means of conventional mixing, dissolving, granulating, dragee-making, levigating, emulsifying, encapsulating, entrapping or lyophilising processes).

The neutron capture compounds of embodiments of the invention can be further characterised as follows. Thus, the compounds comprise an insoluble inorganic material or a mostly inorganic material that contains a neutron capture element selected from Boron, preferably $^{10}$B.

Boron can be employed in embodiments of the invention as elemental Boron or as part of a number of compounds. Elemental Boron, atomic No. 5, atomic weight 10.8 is a black solid at 298k with a density of 2460 kg/m$^3$ and molar volume 4.39 cm$^3$. Boron is available commercially and the most common sources of Boron are tourmaline, borax $[Na_2B_4O_5(OH)_4.8H_2O]$, and kernite $[Na_2B_4O_5(OH)_4.2H_2O]$. In general properties of boron-rich solids such as binary and ternary borides (e.g. $MeB_{12}$, $MeB_{66}$ (Na,Mg,Ln)$MeB_{14}$, the borides of transition elements in beta-rhombohedral boron, boron carbides are structurally similar and can be used for NCT via the particulate route.

Small amounts of high purity boron can be made through the thermal decomposition of compounds such as $BBr_3$ with hydrogen gas using a heated tantalum wire. Results are better with hot wires at temperatures over 1000°C.

Compounds of Boron exhibit a close relationship with respect to crystal structures because they contain B12 icosahedra or related aggregates of atoms. Since these icosahedra determine the electronic structure and hence chemical bonding, there is a very large similarity of chemical and physical properties. Such boron compounds are classified as "boron rich solids". In essence all "boron rich solids" may be used for NCT via the particulate route. In general properties of boron-rich solids such as binary and ternary borides (e.g. $MeB_{12}$, $MeB_{66}$ (Na,Mg,Ln)$MeB_{14}$, the borides of transition elements in beta-rhombohedral boron and boron carbides are structurally similar and exhibit the following properties:

(i) high melting points 2000 - 4000 K; e.g. boron carbide melts at about 2,900 K
(ii) great hardness: at ambient temperatures 2000 - 4500 kp/mm$^2$. Beta-rhombohedral boron is the hardest elementary crystal after diamond, and boron carbide is the third hardest solid after diamond and cubic boron nitride.
(iii) low density e.g. boron carbide has a density of 2.5 gcm$^{-3}$
(iv) small thermal extension coefficient: e.g. boron carbide a = 5.73 10$^{-6}$ K$^{-1}$
(v) high resistance to chemical attack, hence low corrosivety
(vi) high neutron absorption cross section, caused by the $^{10}$B isotope (enrichment in natural B about 20 %)
(vii) semiconducting behavior.

Other well know inert stable borides include Aluminium Boride, Arsenic Boride, Barium Boride , Berrylium Boride, Boron Carbide, Boron Cloride ,Boron Iodine Boron Nitride, Boron Oxide, Boron Zinc, Calcium Boride, Cerium Boride, Chromium Boride, Cobalt Boride, Copper Boride, Diborane Dysporsium Boride, Erbium Boride, Europium Boride, Fermium Boride, Gadolinium Boride, Gold -Boron, Halfnium Boride, Holmium Boride, Iridium Boride, Iron Boride, Lanthanum Boride, Lithium Boride, Lutetium Boride, Magnesium Boride, Manganese Boride, Molybdenum Boride, Neodimium Boride, Neptunium Boride, Nickel Boride, Niobium Boride, Osmium Boride, Palladium Boride, Promethium Boride, Praseodymium Boride, Platinum Boride, Plutonium Boride, Rhenium Boride, Rhodium Boride, Ruthenium Boride, Sulfur Boride, Samarium Boride, Scandium Borid, Silicon Boride, Silver Boride, Strontium Boride, Tantalum Boride, Terbium Boride, Technetium Boride, Thorium Boride, Titanium Boride, Thulium Boride, Tungsten Boride, Uranium Boride, Vanadium Boride, Yttrium Boride, Yitterbium Boride, Zirconium Boride, all of which can also be used for NCT via the particulate route.

In some preferred embodiments, nanoparticles of the invention comprise Boron Carbides which are inert solids with a density of $2.4 \times 10^3$ kg/m$^3$ and a melting point 2450$\underline{o}$C ( K A Schwetz, J Less Common Metals, 82 (1981) 37), Boron rich carbides (M Bouchacourt, J Less Common Metals, 82 (1981) 219) and other carbides including $C_2Al_3B_{48}$ (P.Peshev, J Less Common Metals, 117 (1986) 341) may also be used. In other embodiments, nanoparticles of the invention may comprise BN which is a very inert solid with a density of 3.48,g/cm$^2$ and a melting point of 2730$\underline{o}$C. TiB$_2$ is an extremely hard material with a density of 4.52g/cm3 and may be used in some embodiments of the invention. Boron Oxide which has a density of $2.4 \times 10^3$ kg/m$^3$, and ZrB$_2$ which has a density 6.11 g/cm$^3$ may also be used. In another preferred embodiment, nanoparticles of the invention may comprise $B_{10}H_{14}$ decaborane (14) (boron hydride) decaboron tetradecahydride), a white crystalline solid having a melting point of 213°C, and a density of 950 kg m$^3$. Other hydrides which can be used include B2H6, B4H10, B5H9, B5H11, B6H10.

In addition to the above, the neutron capture compound may take any of the following forms:

(i) neutron capture element as physical solid or a gel-like particle;

(ii) $^{10}B_xM_n$ where M is a metal but can be a non metal such as Nitrogen, Carbon, Oxygen, Chlorine, Bromine or Fluorine;

(iii) $^{10}BxO_n$;

(iv) $^{10}B_xH_n$;

(v) R-$^{10}B_n$ -O$_n$ where R =hydrocarbon chain or other organic chain; or

(vi) (X-O-X) n where X is the neutron capture element.

The overall particle size of the nanoparticles of embodiments of the invention can be about 1Å to 10,000 Å ($10^{-10}$ to $10^{-6}$ m), for example, 1,000 Å to 4,000 Å ($10^{-7}$ to $4 \times 10^{-7}$ m) preferably about 1Å to about 1000 Å ($10^{-10}$ to $10^{-7}$ m), but ideally about 10Å to about 100Å ($10^{-9}$ to $10^{-8}$ m). This range of particle sizes are particularly advantages for a number of reasons. Firstly, particles of this size can be excreted by the kidney and do not remain in the body for a prolonged period of time. Moreover, the particles do not adversely precipitate out in the alveoli of the lungs.

Secondly, particles in this range accumulate in tumours via the "enhanced permeability retention" effect (EPR mechanism) and are able to easily permeate the leaky vasculature of a tumour and become entrapped leading to a build up of a high concentration in the intracellular space. Tumours have a negative osmotic pressure, and have no lymphatic drainage. The vast majority of compounds enter into the tumour via diffusion and tend to remain in the tumour bed and move between cells through gap junctions. NCT compounds normally enter the cell by endocytosis into the cytoplasm and end up in the lysosomal compartment. It is important to note that the NCT compounds need to be targeted such that one or more of the following components of a tumour cell becomes a site of selective disruption that will lead to cell death: the cell membrane, mitochondria, endoplasmic reticulum, golgi apparatus or nucleus.

As well as accumulating in tumour tissues, it has been found that the nanoparticles of embodiments of the invention become internalised in the tumour cells via endocytosis and bring the NCT agent close to the nucleus which can result in more effective killing of tumour cells. Additionally, particles in the 1Å-100Å ($10^{-10}$ to $10^{-8}$ m) range are particularly preferred because they can travel between adjacent cells though "gap junctions", thus providing an important means of distribution to non vascularised parts of the tumour. This process generates a homogeneous distribution of the neutron capture element throughout the tumour mass thereby providing for effective NCT.

For a biologically effective BNCT reaction to take place there is a minimum requirement for the number of boron atoms at the desired location. If boron is located to the outside of a cell (more precisely on the cell membrane) then there should be at least $10^9$ boron atoms for an optimal reaction. If the boron atoms are located inside the cell, say in the cytoplasm then one order of magnitude less Boron atoms are required i.e. $10^8$ and if the $^{10}B$ atoms are in the nucleus of a cell then a further one order of magnitude less $^{10}B$ atoms are required. This negative concentration gradient towards the cell nucleus is due to the fact that the probability of resultant "alpha and lithium" particles damaging the DNA increases with a decrease in the distance between the nucleus and the n+ $^{10}B$ reaction.

With the known compounds BPA, BSH and CuTCPH the number of boron atoms carried is 1, 10 and 40 per molecule respectively. Thus, for example, using BPA located at the cell membrane requires $10^9$ molecules, reducing to $10^8$ in the cytoplasm and $10^7$ in the nucleus. When using CuTCPH, $2.5 \times 10^8$ molecules are required at the cell membrane, $2.5 \times 10^7$ in the cytoplasm and $2.5 \times 10^6$ in the nucleus.

Assuming the mass of a 10 Å spherical particle of embodiments of the invention to be $2.46 \times 10^{-24}$ kg, the number of boron atoms can be calculated based on the density of boron ($2460$ kg/m$^3$). Thus, a 10 Å particle contains $2.28 \times 10^{-22}$ moles of boron, equivalent to 140 atoms. Similarly, a 1000 Å particle has a mass of $2.46 \times 10^{-18}$ kg and contains $2.28 \times 10^{-18}$ moles of boron, equivalent to $1.3 \times 10^8$ atoms.

In view of the above calculations it is apparent that only $7 \times 10^7$ boron particles having a diameter of 10 Å are required to achieve an optimum BNCT reaction at the cell surface, $7 \times 10^6$ particles in the cytoplasm are required and $7 \times 10^5$ particles in the nucleus are required.

If the boron particles have a diameter of 1000 Å then only about 1 particle is required at the nucleus or in the cytoplasm and approximately 2 particles are required at the cell surface for optimum BNCT effect.

Example 1 - Manufacture of sol-gel derived metal glass and ceramics

The sol-gel process for generating metal glass and ceramics can be described as follows:

$$M\text{-}(O\text{-}R)_n + H2O \rightarrow _{n-1}(R\text{-}O)\text{-}M\text{-}OH + (H\text{-}OR)$$

$$_{n-1}(R\text{-}O)\text{-}M\text{-}OH + M\text{-}(O\text{-}R)_n \rightarrow _{n-1}(R\text{-}O)\text{-}M\text{-}O\text{-}M\text{-}(O\text{-}R)_n$$

where M is the metal, for example Ti, Zr, Al, N, B, Si and R can be any carbon chain normally of 1-5 carbons.

This is a dynamic polymerisation reaction comprising a wet chemistry system that yields a metal-oxide system from the reaction of metal-alkoxides.

The metal alkoxides are normally ethoxides, propoxides or butoxides. Pentoxides and higher carbon chains can be used, but the reactivity drops off with chain length and viscosity of the liquids.

Controlled hydrolysis is carried out with the metal alkoxides in proportions to yield a final metal oxide of given elemental ratio at room temperature in the presence of small amounts of $H_2O$. A fine precipitate or a gel or a xerogel can be generated via this process. By controlling the rate of hydrolysis (introduction of controlled amount of moisture) a precipitate may be formed of particles of uniform size. The precipitate or gel or xerogel may be sintered at a temperature of from 100°-1000°-C to drive off remaining organic residues and complete the hydrolysis and desification reactions.

This process is suitable for the production of Si B Glass and Ceramic systems; Si B Na Glass and Ceramic systems; Si B Na Zr Glass and Ceramic systems; Si B Na Zr Ti Glass and Ceramic systems; Ti B Glass and Ceramic systems; Zr B Glass and ceramic systems; and Si Ti Zr B Glass and ceramic systems.

Example 2 - Manufacturing by chemical vapour deposition thin-film coatings and particles

Volatile organics such as Boron tri-ethdoxide and Boron tri-isopropoxide or Boron Halogens $BF_3$, $BCl_3$ may be used to deposit a thin film coating or generate spherical particles of the metal oxide by way of controlled introduction of moisture.

Multi-component metaloxide systems were generated using metal alkoxide precursors of Ti, Zr, Al Si, B, Na. Ethoxide, propoxide and butoxides were used to make sol-gel derived glass and glass ceramics (Titanium tetraisopropoxide, Zirconium tetraisopropoxide, Aluminium tripropoxideu, Tetraethoxysilicone, Sodium ethoxide, Boron tetraethoxide, supplied by Sigma-Aldrich). All the materials except tetraethoxysilicone are hygroscopic and were handled using a vacuum line apparatus with dry nitrogen (1-3ppm water).

The tertraethoxysilicate was hydrolysed to which varying amounts of Ti, Zr, B, Na metalloids were added to produce a stable homogenous sols at room temperature.

In this way, the following metal-oxide glasses were made: Si, Na B, Ti Si B, Zr Si B, Ti Zr Si B and Ti Zr Si B Na.

The above glass systems were made by first hydrolysing Si $(OCH_2CH_3)_4$ (TEOS) with ~1.5 moles of water in 0.1 % HCl. Typically, 30g of TEOS, 5g of $H_2O$ and 20ul of 3MHCl in 35g of Proponol and 35g of Ethanol was refluxed for 150 minutes. This process generates a silicate sol-gel (a partially cross linked -Si-O-Si- system) that yields between ~10% $SiO_2$. To this other precursors of the metal oxide glass formers were added to generate the metal oxide glasses. Upon cooling one or more metal oxide precursors of Ti, Zr, Na, B was gently added (3.5g of each precursor) whilst continuously stirring. A clear transparent liquid resulted. The mixture was allowed to stand at room temperature with the flask covered with cellophane with pinholes so as to allow atmospheric moisture in and for slow evaporation of solvents. The slow introduction of atmospheric moisture is necessary to aid hydrolysis of the Ti or Na or Al or Zr or B alkoxides and form a cross-linked glassy structure. After 8-24 hours standing in a fume cupboard a transparent gel results which upon further aging for up to 3-7 days shrinks in to a solid transparent xerogel ( a porous glass). The xerogel was processed using mechanical pulverisation or grinding for 24-28 hours in a motorised ceramic pestle and mortar. Following this the powder was further reduced and a surfactant coating added by way of using a ball-mill.

The xerogel was heated to a temperature of 600C and 1100C to drive off any organic components leaving a black glass containing the respective metal oxides. Again the glass was processed using mechanical pulverisation or grinding for 24-28 hours in a motorised pestle mortar (25-30g grinding capacity). The powder was further reduced in particle size using the ball-mills in example 4.

These mechanical pulverisation process generated fine particles of approximately 0.1-0.5 and 1 um-5um particles.

In some experiments where the reflux times were reduced to 60-120 minutes, post cooling and following the addition of metal oxide precursors of Ti, Zr, Al, B and Na a fine suspension results. The examination of the particles post evaporation of the solvents and 37 C oven drying and desiccation reveals crystalline particles of sub-micron range (typically 0.01 um-1 um with some in the range of 1-5um).

Example 3 - Coating nanoparticles with biocompatible "stealth" materials

At the time of particle grinding the surfaces of the particles were coated with Polyoxyrthlene sorbitan fatty acid diester or Sorbitan ester of a fatty acid with 10 and 20 carbon atoms or polyvinylpyrrolidone (PVP) or Gum acacia. Between 1 % and 10% wt/wt of NCT material.

The surface modifier was diluted in water and a slurry (10% to 30%wt/wt) of the NCT particles was made for particle reduction in a ball-mill. The slurry was milled for 48 hours and resulted in a "milky" stable suspension. Typically, the particles were between 0.01-0.5$\mu$m.

Example 4 - Preparation of nanoparticles

Boron Nitride was used to prepare nanoparticles. Boron Nitride powder of approximately 1$\mu$m average particle size from Aldrich, Product code 255475 was used. Polyvinylpyrrolidone K15 , Molecular weight-10000, from Aldrich, Product code 81390 was used to condition the surface of the particles. The mills were operated for 24-48 hours

Two mills were used to generate nanoparticles as follows:

A conventional mill constructed of porcelain having an internal diameter of 110 mm and a depth of 100 mm. This mill is run at a rotation speed of 1.25 Hz (35% of critical speed). The milling media was made of 10 mm diameter porcelain spheres with a total milling media volume of 500 ml. This mill was used with a mixture of, for example:

| | |
|---|---|
| BN | 7.5 g |
| PVP | 3.0 g |
| Water | ~200 ml |

A customised mill has also been used. This mill was designed not to surge and be good for mixing and attrition which is the most effective mechanism for fine grinding and produces a uniform particle size. The mill has a stainless steel internal diameter of 50 mm and a depth of 50 mm, and operates at a rotation speed of 1.25 Hz (14% of critical speed). The milling media was made of 3 mm diameter stainless steel spheres with a total milling media volume of approximately 50 ml. This mill may be used with a mixture of, for example:

| | |
|---|---|
| Mixture volume (max) | ~50 |
| BN | 2.50 g |
| PVP | 0.75 g |
| Water | 50 ml |

Example 5 - Preparation of nanoparticles by milling in the absence of abrasive

A conventional mill as described above was used to generate Boron Nitride nanoparticles from Boron Nitride powder, except that no milling media was used. This provided for pure Boron Nitride nanoparticles containing no contamination from milling media.

In a further example, the customised mill of the earlier example was also used without adding the milling media to provide pure Boron Nitride nanoparticles.

Example 6 - Experimental evaluation of inorganic nanoparticle systems for BNCT

**Materials and methods**

**Compounds Used**

The following methods may be used to evaluate inorganic nanoparticles of the invention, for example, comprising BN, B, BC or Si Ti B glass particles, with or without surface modification.

**Surface Modification**

The particles may be coated with the following surface modifiers to provide a biocompatible stealth layer as described above: polyoxyrthlene sorbitan fatty acid ester; sorbitan ester of a fatty acid with 10-20 carbon atoms; PVA; and gum acacia.

**Animal numbers and species**

Studies using rodents involve standard, established techniques and statistical methods for data analysis. On average, groups of 8 rats per data point are used in pharmacokinetic, biodistribution and radiobiological studies. The animal models are male Fischer 344 rats (250-300 g), female BALB mice (20-25 g) and SCID mice (20-25 g). In all studies, anaesthesia is maintained with ketamine (120 mg/kg) and xylazine (20 mg/kg). Animals are monitored on a daily basis for general health. Animals are euthanised, as required, under anaesthesia.

**Boron pharmacokinetics, biodistribution and toxicology**

Evaluation of Å sized inorganic nanoparticles takes place using appropriate tumour models. These include glioma (rat 9L gliosarcoma), colon cancer (mouse), melanoma (murine Harding Passey melanoma), breast cancer (murine EMT-6 adenocarcinoma) and prostate cancer (prostate xenograft in SCID mice), all transplanted subcutaneously.

Pharmacokinetic/biodistribution analysis involve the administration of graded doses of a given boronated antibody, with sampling (blood, tumour, normal tissues) at time intervals of up to 6 days after administration. Inorganic particle systems delivering >30 $\mu$g boron/g to the tumour with a tumour:blood boron partition ratio of >3:1 is considered suitable for further evaluation. Studies also involve observations on behavior, changes in body weight, chemical and hematological tests (Miura et al., 1998).

The boron content of relevant normal tissues (skin, oral mucosa, brain, spinal cord and blood) and implanted tumours is measured using direct coupled plasma atomic emission spectrometry (Coderre et al., 1994).

**Radiobiology Studies -Neutron Irradiations**

Standard experimental collimators, irradiation jigs and body shielding are used for irradiations. Standard dosimetric procedures are well established and using gold foils, thermoluminescent dosimeters and ionisation chambers (Coderre et al.,1992; Morris et al., 1994a,b).

**Pre-clinical therapeutic studies**

Experimental evaluation of therapeutic efficacy entail localised irradiation of tumour-bearing animals with graded doses of thermal neutrons in the presence of the boronated antibody. The administration protocols and the timing after administration at which irradiation begins will be determined by prior pharmacokinetic and biodistribution studies. The assessment consist of quantification of changes in tumour volume, incidence of tumour ablation and long-term survival. By way of comparison, and to enable calculation of RBE/CBE factors, graded doses of x-rays and of the thermal beam alone is given.

**Histology**

Standard histopathological analysis is carried out on irradiated normal tissues and tumours using standard techniques. Animals are perfusion-fixed using 10% buffered formal saline.

**Data analysis**

Dose-response data using the various endpoints is assessed using probit analysis, and ED50 and TCP50 values are derived from the fitted curves. These values are used to calculate RBE/CBE factor values. Appropriate control groups are used in all studies. In therapy experiments, the percentage survival versus time following irradiation is analysed using the Kaplan-Meier method. The survival times in each of the experimental groups is ordered and ranked for comparison by a non-parametric statistical technique, Wilcoxin 2-Sample Test.

**Results**

In one experiment a 20%wt/wt suspension of Si Ti B glass particles coated with PVP (prepared in the ball-mall) was assessed for biodistribtuion as described above. An average of $10\mu$g-$25\mu$g boron atoms per g of wet tumour tissue was found 2 days after intravenous administration.

In a further experiment, a 10%, 20% and 30% wt/wt suspension of BN particles coated with PVP were assessed for biodistribution. An average of $15\mu$g-$35\mu$g boron atoms per g of wet tumour tissue was found 2 days after intravenous administration.

When normal tissue was measured less than 0.1 $\mu$g/g boron atoms were found in skin, whilst 1 to 5 $\mu$g/g boron atoms were found in liver and lung, demonstrating accumulation of boron preferentially in tumour tissue.

Throughout the specification, various different embodiments have been described for a number of features of the invention including, but not limited to, the physical form and size of the nanoparticles, the identity and nature of the neutron capture element and further metals to be included in the inorganic nanoparticles, uses of the nanoparticles and the presence or absence and nature of a biocompatible stealth layer as well as other ingredients in the nanoparticles. The application is intended to cover all possible combinations of each of these features and any other features provided as part of a list except where it would be apparent to the skilled person that such combinations were self-contradictory. In particular, each or any of the physical form and sizes of nanoparticles are intended to be combined individually or together with any neutron capture element in any form, and any such nanoparticles are suitable for any of the uses disclosed. Any such nanoparticle formed of any combination of the above is intended to be used with any of the biocompatible stealth layers disclosed or with no biocompatible stealth layer. Any other, and all suitable combinations will occur to the skilled reader and the Examples given are not intended to be limiting on the scope of the invention.

**Claims**

1. A water insoluble nanoparticle comprising at least one neutron capture element in an inorganic form for use in therapy, surgery or diagnosis wherein said at least one neutron capture element is selected from the group consisting of boron, preferably $^{10}$B, said nanoparticle comprising a biocompatible outer layer selected from polyvinylpyrrolidine (PVP).

2. A nanoparticle as claimed in claim 1 having a particle size of about $10^{-10}$ m to about $10^{-6}$ m, preferably having a particle size of about $10^{-10}$ m to about $10^{-7}$ m, more preferably having a particle size of about $10^{-9}$ m to about $10^{-8}$ m.

3. A nanoparticle as claimed in claim 1 or claim 2 wherein said neutron capture element is in its natural crystalline form, in a particular form, in the form of a glass or
   a glass ceramic, in the form of a polymerised inorganic matrix, in the form of a sol-gel derived xerogel.

4. A nanoparticle as claimed in any of claims 1 to 3 wherein said neutron capture element is in the form of an organically modified ceramic and wherein the element comprises at least one bond to a hydrocarbon chain.

5. A nanoparticle as claimed in claim 1 wherein said boron is in the form of:

   (i) $^{10}B_xM_n$;
   (ii) $^{10}B_xH_n$; or
   (iii) R-$^{10}B_n$-$O_n$

   wherein M is a metal or is selected from nitrogen, carbon, oxygen, chlorine, bromine or fluorine, x and n are integers of one or above and R is a hydrocarbon chain or other organic chain.

6. A nanoparticle as claimed in claim 1 wherein said neutron capture element is in the form of $(X-O-X)_n$ wherein n is

an integer of 1 or above and X is the neutron capture element.

7. A nanoparticle as claimed in any preceding claim wherein the neutron capture element is present as a layer or film around an inorganic nanoparticle core.

8. A nanoparticle as claimed in claim 7 wherein said core is selected from mica, zeolites, $TiO_2$ spheres, $ZrO_2$ spheres or particle or organic polymer particles or spheres.

9. A nanoparticle as claimed in any preceding claim wherein said particles further comprise a pharmacologically active substance.

10. A nanoparticle as claimed in claim 9 wherein said pharmacologically active substance is loaded into said nanoparticles by absorption, adsorption or incorporation.

11. A nanoparticle as claimed in claim 9 or claim 10 wherein said pharmacologically active substance is a chemotherapeutic agent.

12. A nanoparticle as claimed in any preceding claim further comprising a further metal selected from the group consisting of vanadium (V), manganese (Mn), iron (Fe), ruthenium (Ru), technetium (Tc), chromium (Cr), platinum (Pt), cobalt (Co), nickel (Ni), copper (Cu), zinc (Zn), germanium (Ge) indium (In), tin (Sn), yttrium (Y), gold (Au), barium (Ba), tungsten (W) and gadolinium (Gd).

13. A nanoparticle as claimed in claim 12 wherein said further metal is present at a concentration of about 0.000 1 % wt/wt to about 0.1 % wt/wt.

14. A pharmaceutical composition comprising a water insoluble nanoparticle comprising at least one neutron capture element in an inorganic form according to any one of claims 1 to 13.

15. The use of water insoluble nanoparticles comprising at least one neutron capture element in an inorganic form as defined in any one of claims 1 to 21 in the manufacture of a medicament for use in neutron capture therapy.

16. A use as claimed in claim 15 wherein said neutron capture therapy is for the , treatment or ablation of cancer or other diseased tissues e.g. lymphomas, skin cancer, breast cancer, lung cancer, head and neck cancer, bone cancer, prostate cancer, cancer of the pancreas, cervical cancer, brain cancer e.g. glioblastomas, primary and secondary metastases and benign and metastatic prostate cancers, e.g. benign prostate hyperplasia.

17. A use as claimed in claim 16 wherein said tumour is solid and discrete.

18. A use as claimed in any one of claims 15 to 17 wherein said neutron capture therapy is administered over a period of one to fourteen days.

19. A process for the preparation of water insoluble nanoparticles comprising at least one neutron capture element in an inorganic form as claimed in any one of claims 1 to 21, said process comprising:

    (i) providing at least a first mass of said neutron capture element in an inorganic form;
    (ii) providing at least a second mass of the same type of material;
    (iii) mixing said first and second masses in the absence of other abrasive material;
    (iv) causing frictional abrasion between said first and second masses; and
    (v) collecting said nanoparticles.

20. A process as claimed in claim 19 wherein at least one further, non abrasive material is included in said mixing step (iii).

21. A process as claimed in claim 20 wherein said further, non-abrasive material is selected from the group consisting of vanadium (V), manganese (Mn), iron (Fe), ruthenium (Ru), technetium (Tc), chromium (Cr), platinum (Pt), cobalt (Co), nickel (Ni), copper (Cu), zinc (Zn), germanium (Ge) indium (In), tin (Sn), yttrium (Y), gold (Au), barium (Ba), tungsten (W) and gadolinium (Gd).

22. A process as claimed in any one of claims 19 to 21 wherein the resulting nanoparticles are **characterised by** any of the features of claims 1 to 18.

**Patentansprüche**

1. Wasserunlösliches Nanopartikel, umfassend mindestens ein Neutroneneinfangelement in einer anorganischen Form, zur Verwendung in Therapie, Chirurgie oder Diagnose, wobei besagtes mindestens eine Neutroneneinfangelement ausgewählt ist aus der Gruppe bestehend aus Bor, vorzugsweise $^{10}B$, wobei besagtes Nanopartikel eine biokompatible äußere Schicht umfasst, ausgewählt aus Polyvinylpyrrolidin (PVP).

2. Nanopartikel wie in Anspruch 1 beansprucht mit einer Partikelgröße von ungefähr $10^{-10}$ m bis ungefähr $10^{-6}$ m, vorzugsweise mit einer Partikelgröße von ungefähr $10^{-10}$ m bis ungefähr $10^{-7}$ m, bevorzugter mit einer Partikelgröße von ungefähr $10^{-9}$ m bis ungefähr $10^{-8}$ m.

3. Nanopartikel wie in Anspruch 1 oder Anspruch 2 beansprucht, worin besagtes Neutroneneinfangelement seine natürliche kristalline Form, eine partikuläre Form, die Form eines Glases oder einer Glaskeramik, die Form einer polymerisierten anorganischen Matrix, die Form eines Sol-Gel-abgeleiteten Xerogels hat.

4. Nanopartikel wie in einem von Ansprüchen 1 bis 3 beansprucht, worin besagtes Neutroneneinfangelement die Form einer organisch modifizierten Keramik hat und worin das Element mindestens eine Bindung an eine Kohlenwasserstoffkette umfasst.

5. Nanopartikel wie in Anspruch 1 beansprucht, worin besagtes Bor die Form von:

   (i) $^{10}B_xM_n$;
   (ii) $^{10}B_xH_n$; oder
   (iii) $R\text{-}^{10}B_n\text{-}O_n$

   hat, worin M ein Metall darstellt oder ausgewählt ist aus Stickstoff, Kohlenstoff, Sauerstoff, Chlor, Brom oder Fluor, x und n ganze Zahlen von eins oder darüber sind und R für eine Kohlenwasserstoffkette oder andere organische Kette steht.

6. Nanopartikel wie in Anspruch 1 beansprucht, worin besagtes Neutroneneinfangelement die Form von $(X\text{-}O\text{-}X)_n$ hat, worin n eine ganze Zahl von 1 oder darüber ist und X das Neutroneneinfangelement darstellt.

7. Nanopartikel wie in einem vorangehenden Anspruch beansprucht, worin das Neutroneneinfangelement als Schicht oder Film um einen anorganischen Nanopartikelkern herum vorhanden ist.

8. Nanopartikel wie Anspruch 7 beansprucht, worin besagter Kern ausgewählt ist aus Mika, Zeolithen, $TiO_2$-Kugeln, $ZrO_2$-Kugeln oder -Partikel oder organischen Polymerpartikeln oder -kugeln.

9. Nanopartikel wie in einem vorangehenden Anspruch beansprucht, wobei besagte Partikel ferner eine pharmakologisch aktive Substanz umfassen.

10. Nanopartikel wie in Anspruch 9 beansprucht, wobei besagte pharmakologisch aktive Substanz durch Absorption, Adsorption oder Einschließen in besagte Nanopartikel geladen ist.

11. Nanopartikel wie in Anspruch 9 oder Anspruch 10 beansprucht, worin besagte pharmakologisch aktive Substanz ein chemotherapeutischer Wirkstoff ist.

12. Nanopartikel wie in einem vorangehenden Anspruch beansprucht, welches ferner ein weiteres Metall umfasst, ausgewählt aus der Gruppe bestehend aus Vanadium (V), Mangan (Mn), Eisen (Fe), Ruthenium (Ru), Technetium (Tc), Chrom (Cr), Platin (Pt), Cobalt (Co), Nickel (Ni), Kupfer (Cu), Zink (Zn), Germanium (Ge), Indium (In), Zinn (Sn), Yttrium (Y), Gold (Au), Barium (Ba), Wolfram (W) und Gadolinium (Gd).

13. Nanopartikel wie in Anspruch 12 beansprucht, worin besagtes weiteres Metall bei einer Konzentration von ungefähr 0,0001% Gew./Gew. bis ungefähr 0,1% Gew./Gew. vorhanden ist.

14. Pharmazeutische Zusammensetzung, welche ein wasserunlösliches Nanopartikel, umfassend mindestens ein Neutroneneinfangelement in einer anorganischen Form gemäß einem von Ansprüchen 1 bis 13, umfasst.

15. Verwendung von wasserunlöslichen Nanopartikeln, welche mindestens ein Neutroneneinfangelement in einer anorganischen Form wie in einem von Ansprüchen 1 bis 21 definiert umfassen, in der Herstellung eines Medikaments zur Verwendung in Neutroneneinfangtherapie.

16. Verwendung wie in Anspruch 15 beansprucht, wobei besagte Neutroneneinfangtherapie für die Behandlung oder Ablation von Krebs oder anderen erkrankten Geweben z.B. Lymphomen, Hautkrebs, Brustkrebs, Lungenkrebs, Kopf- und Nackenkrebs, Knochenkrebs, Prostatakrebs, Krebs der Bauchspeicheldrüse, Gebärmutterhalskrebs, Gehirnkrebs z.B. Glioblastomen, primären und sekundären Metastasen und gutartigen und metastatischen Prostatakrebsen, z.B. gutartiger Prostatahyperplasie ist.

17. Verwendung wie in Anspruch 16 beansprucht, wobei besagter Tumor fest und abgesondert ist.

18. Verwendung wie in einem von Ansprüchen 15 bis 17 beansprucht, wobei besagte Neutroneneinfangtherapie über einen Zeitraum von einem bis vierzehn Tagen verabreicht wird.

19. Verfahren zur Herstellung von wasserunlöslichen Nanopartikeln, umfassend mindestens ein Neutroneneinfangelement in einer anorganischen Form wie in einem von Ansprüchen 1 bis 21 beansprucht, wobei besagtes Verfahren umfasst:

(i) Vorsehen von mindestens einer ersten Masse von besagtem Neutroneneinfangelement in einer anorganischen Form;
(ii) Vorsehen von mindestens einer zweiten Masse des gleichen Materialtyps;
(iii) Mischen der besagten ersten und zweiten Massen in Abwesenheit von anderem Schleifmaterial;
(iv) Verursachen von Reibungsabrieb zwischen besagten ersten und zweiten Massen; und
(v) Sammeln von besagten Nanopartikeln.

20. Verfahren wie in Anspruch 19 beansprucht, wobei mindestens ein weiteres, abriebfestes Material in besagtem Mischschritt (iii) eingeschlossen ist.

21. Verfahren wie in Anspruch 20 beansprucht, wobei besagtes weiteres, abriebfestes Material ausgewählt ist aus der Gruppe bestehend aus Vanadium (V), Mangan (Mn), Eisen (Fe), Ruthenium (Ru), Technetium (Tc), Chrom (Cr), Platin (Pt), Cobalt (Co), Nickel (Ni), Kupfer (Cu), Zink (Zn), Germanium (Ge), Indium (In), Zinn (Sn), Yttrium (Y), Gold (Au), Barium (Ba), Wolfram (W) und Gadolinium (Gd).

22. Verfahren wie in einem von Ansprüchen 19 bis 21 beansprucht, wobei die sich ergebenden Nanopartikel durch eines der Merkmale von Ansprüchen 1 bis 18 **gekennzeichnet** sind.

## Revendications

1. Nanoparticule insoluble dans l'eau comprenant au moins un élément de capture de neutrons sous une forme inorganique pour une utilisation en thérapie, chirurgie ou diagnostic où ledit au moins un élément de capture de neutrons est choisi dans le groupe constitué de bore, de préférence $^{10}$B, ladite nanoparticule comprenant une couche externe biocompatible choisie parmi la polyvinylpyrrolidine (PVP).

2. Nanoparticule selon la revendication 1, ayant une taille de particule d'environ $10^{-10}$ m à environ $10^{-6}$ m, de préférence ayant une taille de particule d'environ $10^{-10}$ m à environ $10^{-7}$ m, de manière davantage préférée ayant une taille de particule d'environ $10^{-9}$ m à environ $10^{-8}$ m.

3. Nanoparticule selon la revendication 1 ou la revendication 2, dans laquelle ledit élément de capture de neutrons est sous sa forme cristalline naturelle, sous une forme particulière, sous la forme d'un verre ou d'une céramique vitreuse, sous la forme d'une matrice inorganique polymérisée, sous la forme d'un xérogel dérivé de sol-gel.

4. Nanoparticule selon l'une quelconque des revendications 1 à 3, dans laquelle ledit élément de capture de neutrons est sous la forme d'une céramique modifiée de manière organique et où l'élément comprend au moins une liaison à une chaîne d'hydrocarbure.

5. Nanoparticule selon la revendication 1, où ledit bore est sous la forme de :

    (i) $^{10}B_xM_n$;
    (ii) $^{10}B_xH_n$ ; ou
    (iii) $R\text{-}^{10}B_n\text{-}O_n$

où M est un métal ou est choisi parmi l'azote, le carbone, l'oxygène, le chlore, le brome ou le fluor, x et n sont des nombres entiers de un ou supérieurs et R représente une chaîne d'hydrocarbure ou une autre chaîne organique.

6. Nanoparticule selon la revendication 1, dans laquelle ledit élément de capture de neutrons est sous la forme de $(X\text{-}O\text{-}X)_n$ où n est un nombre entier de 1 ou supérieur et X est l'élément de capture de neutrons.

7. Nanoparticule selon l'une quelconque des revendications précédentes, dans laquelle l'élément de capture de neutrons est présent sous la forme d'une couche ou d'un film autour d'un noyau nanoparticulaire inorganique.

8. Nanoparticule selon la revendication 7, dans laquelle ledit noyau est choisi parmi du mica, des zéolites, des sphères de $TiO_2$, des sphères ou des particules de $ZrO_2$ ou des particules ou des sphères de polymères organiques.

9. Nanoparticule selon l'une quelconque des revendications précédentes, où lesdites particules comprennent en outre une substance pharmacologiquement active.

10. Nanoparticule selon la revendication 9, où ladite substance pharmacologiquement active est chargée dans lesdites nanoparticules par absorption, adsorption ou incorporation.

11. Nanoparticule selon la revendication 9 ou la revendication 10, où ladite substance pharmacologiquement active est un agent chimiothérapeutique.

12. Nanoparticule selon l'une quelconque des revendications précédentes comprenant en outre un autre métal choisi dans le groupe constitué de vanadium (V), manganèse (Mn), fer (Fe), ruthénium (Ru), technétium (Tc), chrome (Cr), platine (Pt), cobalt (Co), nickel (Ni), cuivre (Cu), zinc (Zn), germanium (Ge), indium (In), étain (Sn), yttrium (Y), or (Au), baryum (Ba), tungstène (W) et gadolinium (Gd).

13. Nanoparticule selon la revendication 12, dans laquelle ledit autre métal est présent à une concentration d'environ 0,0001 % p/p à environ 0,1 % p/p.

14. Composition pharmaceutique comprenant une nanoparticule insoluble dans l'eau comprenant au moins un élément de capture de neutrons sous une forme inorganique selon l'une quelconque des revendications 1 à 13.

15. Utilisation de nanoparticules insolubles dans l'eau comprenant au moins un élément de capture de neutrons sous une forme inorganique telles que définies dans l'une quelconque des revendications 1 à 13 dans la fabrication d'un médicament pour une utilisation en thérapie par capture de neutrons.

16. Utilisation selon la revendication 15, où ladite thérapie par capture de neutrons est destinée au traitement ou à l'ablation d'un cancer ou d'autres tissus malades, par exemple, des lymphomes, un cancer de la peau, un cancer du sein, un cancer du poumon, un cancer de la tête et du cou, un cancer des os, un cancer de la prostate, un cancer du pancréas, un cancer du col de l'utérus, un cancer du cerveau, par exemple, des glioblastomes, des métastases primaires et secondaires et des cancers de la prostate bénins et métastasés, par exemple, une hyperplasie bénigne de la prostate.

17. Utilisation selon la revendication 16, où ladite tumeur est solide et discrète.

18. Utilisation selon l'une quelconque des revendications 15 à 17, où ladite thérapie par capture de neutrons est administrée sur une période d'un à quatorze jours.

19. Procédé de préparation de nanoparticules insolubles dans l'eau comprenant au moins un élément de capture de neutrons sous une forme inorganique selon l'une quelconque des revendications 1 à 21, ledit procédé comprenant :

(i) la fourniture d'au moins une première masse dudit élément de capture de neutrons sous une forme inorganique ;
(ii) la fourniture d'au moins une seconde masse du même type de matériau ;
(iii) le mélange desdites première et seconde masses en l'absence en l'absence d'autre matériau abrasif ;
(iv) la provocation d'une abrasion par friction entre lesdites première et seconde masses ; et
(v) le recueil desdites nanoparticules.

20. Procédé selon la revendication 19, dans lequel au moins un autre matériau non abrasif est inclus dans ladite étape de mélange (iii).

21. Procédé selon la revendication 20, dans lequel ledit autre matériau non abrasif est choisi dans le groupe constitué de vanadium (V), manganèse (Mn), fer (Fe), ruthénium (Ru), technétium (Tc), chrome (Cr), platine (Pt), cobalt (Co), nickel (Ni), cuivre (Cu), zinc (Zn), germanium (Ge), indium (In), étain (Sn), yttrium (Y), or (Au), baryum (Ba), tungstène (W) et gadolinium (Gd).

22. Procédé selon l'une quelconque des revendications 19 à 21, dans lequel les nanoparticules résultantes sont **caractérisées par** l'un quelconque des caractères des revendications 1 à 18.

Figure 1

A

B

Figure 2

Figure 3

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5846741 A, Griffiths G L

### Non-patent literature cited in the description

- **Snyder H R et al.** *J Am Chem Soc,* 1958, vol. 80, 835
- **Soloway.** *Chem Rev.,* 1998, 1531-1534
- **Hawthorn, M F et al.** *J Am Chem Soc,* 1959, vol. 81, 5519
- **Grimes R.** N. Carboranes. Academic press, 1970
- **Soloway A H et al.** *J Med Chem,* 1967, vol. 10, 714
- **Ozawa T.** *Pro Am Ass for Cancer,* March 1998, vol. 39, 586
- **Miura M.** *Radiation research,* 2001, vol. 155, 603-610
- **Liao. T.K.** *J Am Chem Soc,* 1964, vol. 86, 1869
- **Schinazi R, F.** *J Org. Chem. Soc,* 1985, vol. 50, 841
- **Nemoto. H.** *J Chem Soc. Chem Commun.,* 1994, 577
- **Soloway.** *Chemical reviews,* 1998, vol. 98 (4), 1538-1541
- **Soloway.** *Chemical reviews,* 1998, vol. 98 (4), 1519
- **Soloway.** *Chemical reviews,* 1998, vol. 98 (4), 1533-1534
- **Shelly K.** *Proc Natl Acad Sci USA,* 1992, vol. 89, 9039
- **Chou Fl.** *Anticancer Res,* May 1999, vol. 19 (3A), 1759-64
- **Soloway.** *Chemical reviews,* 1998, vol. 98 (4), 1545-1550
- **K A Schwetz.** *J Less Common Metals,* 1981, vol. 82, 37
- **M Bouchacourt.** *J Less Common Metals,* 1981, vol. 82, 219
- **P.Peshev.** *J Less Common Metals,* 1986, vol. 117, 341